# EUROPÄISCHE PATENTSCHRIFT

(11) **EP 2 032 088 B1**
(45) Veröffentlichungstag und Bekanntmachung des Hinweises auf die Patenterteilung: **19.08.2015**
(21) Anmeldenummer: 07726155.0
(22) Anmeldetag: 26.06.2007
(51) Int. Cl.: A61F 2/46

(54) **EINSETZINSTRUMENT FÜR GELENKPFANNEN VON PROTHESEN**
INSERTION INSTRUMENT FOR JOINT SOCKETS OF PROSTHESES
INSTRUMENT D'INSERTION POUR CAVITÉS GLÉNOÏDES DE PROTHÈSES

(30) Priorität: 28.06.2006 DE 202006010069 U
(43) Veröffentlichungstag der Anmeldung: 11.03.2009
(73) Patentinhaber: Waldemar Link GmbH & Co., 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2007/005647
(87) Internationale Veröffentlichungsnummer: WO 2008/000442

(56) Entgegenhaltungen:
- DE-A1- 19 704 577
- DE-A1- 19 722 923
- DE-C1- 19 824 328
- FR-A- 2 797 180
- FR-A- 2 809 305

## Beschreibung

Die Erfindung betrifft ein Einsetzinstrument für Gelenkpfannen von Prothesen mit einem lang gestrecktem Schaft, an dessen vorderen Ende ein Saugkopf zur Verbindung mit der Gelenkpfanne und an dem weiter eine Saugeinrichtung mit einem in dem Schaft geführten Kolben, einer am Saugkopf mündenden Saugleitung und einem Betätigungsorgan angeordnet ist, das entlang einer Führungsbahn am Schaft geführt ist.

Prothesen für bestimmte Gelenke des menschlichen Körpers, insbesondere für Schulter- und Hüftgelenke, weisen in der Regel eine Komponente auf, die als eine Gelenkpfanne ausgebildet ist. Bei einer Hüftprothese handelt es sich hierbei meist um die in das Acetabulum einzusetzende Komponente. Sie kann einteilig oder zweiteilig mit einem Außenträger und einem gesonderten, in diesen einzusetzenden Lagereinsatz ausgeführt sein. Bei letztgenannter Ausführung ergibt sich das Problem, dass die Befestigung bei zementfreier Implantation ein Einschlagen der Komponente erfordert. Hierbei kommt es leicht zu Beschädigungen an dem empfindlichen Lagereinsatz, insbesondere wenn dieser aus Kunststoff oder Keramikmaterial besteht. Es besteht also Bedarf an einem Instrument, mit dem die Handhabung der Gelenkpfanne beim Einsetzen, insbesondere beim Einschlagen, erleichtert ist.

Aus der US-A-3 859 992 ist ein Instrument bekannt, das eine vakuumbetätigte Pfannenhalterung aufweist. Das Instrument weist am vorderen Ende des lang gestreckten Schafts einen Saugkopf auf, über den mittels eines rohrartigen Anschlussstücks mit einem Lüftungsventil eine Vakuumquelle anschließbar ist. Die zu implantierende Gelenkpfanne wird auf dem Saugkopf aufgesteckt, die im Saugkopf mündende Leitung mit einem Unterdruck beaufschlagt und so die Gelenkpfanne an dem Saugkopf gehalten. Die Gelenkpfanne kann nun eingesetzt werden, und gegebenenfalls mittels eines am anderen Ende des Schafts angeordneten Schlagkopfs eingeschlagen werden. Zum Abnehmen des Instruments wird mittels des Belüftungsventils die Saugleitung belüftet, wodurch das Vakuum in dem Saugkopf zusammenbricht und das Instrument mit dem Saugkopf leicht von der eingeschlagenen Gelenkpfanne abgenommen werden kann. Die Anschlussleitung ist gesondert von dem Schaft ausgeführt, und steht V-artig von ihr ab. Das Instrument ist damit verhältnismäßig ausladend und von der Handhabung gerade bei beengten Operationsverhältnissen ungünstig.

Aus der DE-A-197 22 923 ist ein weiter entwickeltes Instrument bekannt. Es weist ebenfalls einen Schaft mit einem Saugkopf am vorderen und einem Schlagkopf am hinteren Ende auf. Ferner ist eine in dem Saugkopf mündende Saugleitung vorgesehen, die mit Unterdruck beaufschlagbar ist. Jedoch wird der Unterdruck nicht durch das Anschließen einer externen Vakuumquelle bereitgestellt, sondern durch eine in das Instrument integrierte Pumpe erzeugt. Dazu ist eine in den Schaft integrierte Kolbenpumpe vorgesehen. Ein in Längsrichtung verschiebbarer Kolben wird mittels einer quer durch den Kolben geschobenen Betätigungsstange nach hinten bewegt, wodurch ein Unterdruck durch ein- oder mehrmalige Betätigung erzeugt wird. Ein Belüftungsventil verhindert in seinem geschlossenen Zustand, dass das Vakuum entweicht. Damit kann, wie bereits zu vorstehendem Instrument näher beschrieben, die Gelenkpfanne sicher an dem Instrument gehalten werden und an dem vorgesehenen Ort eingesetzt bzw. eingeschlagen werden. Zum Abschluss wird das Belüftungsventil geöffnet, wodurch der Unterdruck entweichen und das Instrument leicht von der Gelenkpfanne abgenommen werden kann. Die integrierte Kolbenpumpe stellt zwar eine Verbesserung gegenüber den aus der US-A-3 859 992 bekanntem Instrument dar, jedoch ist die Handhabung noch verhältnismäßig kompliziert. Zudem erhält der Benutzer abgesehen von der Stellung des Belüftungsventils keine Information darüber, ob die Saugleitung ausreichend mit Unterdruck beaufschlagt ist oder nicht.

Der Erfindung liegt ausgehend von dem zuletzt genannten Stand der Technik daher die Aufgabe zu Grunde, ein Instrument der eingangs genannten Art dahingehend zu verbessern, dass es eine sicherere und einfachere Handhabung ermöglicht.

Die erfindungsgemäße dieser Lösung liegt in einem Instrument mit den Merkmalen des unabhängigen Anspruchs. Vorteilhafte Weiterbildungen sind Gegenstand des abhängigen Anspruchs.

Erfindungsgemäß ist bei einem Einsetzinstrument für Gelenkpfannen von Prothesen mit einem lang gestrecktem Schaft, an dessen vorderem Ende ein Saugkopf zur Verbindung mit der Gelenkpfanne vorgesehen ist, und einer Saugeinrichtung mit einem im Schaft geführten Kolben, einer am Saugkopf mündenden Saugleitung und einem Betätigungsorgan, das entlang einer Führungsbahn am Schaft geführt ist, vorgesehen, dass die Führungsbahn helixartig geformt ist und eine Arretierungseinrichtung für eine Saugposition aufweist.

Durch die helixartige Form wird erreicht, dass das Betätigungsorgan während des Bewegens des Kolbens in seine Saugposition schraubenförmig nach hinten bewegt wird. Dabei führt das Betätigungsorgan sowohl eine Drehbewegung wie auch eine Längsbewegung aus. Durch die Längsbewegung wird in dem Saugkanal ein Unterdruck zum Halten der Gelenkpfanne an dem Saugkopf erzeugt. Durch die Drehbewegung wird das Betätigungsorgan dabei gleichzeitig soweit verdreht, bis es am Ende der Führungsbahn eine Arretierposition erreicht. In dieser ist das Betätigungsorgan stabil gehaltert. Vorzugsweise ist die Arretierposition als eine Vertiefung ausgeführt. Dabei macht die veränderte Winkelposition des Betätigungsorgans es dem Operateur möglich, auf einen Blick von hinten zu erkennen, ob sich der Kolben mit dem Betätigungsorgan in der hinteren, arretierten Stellung befindet und damit Unterdruck aufgebaut ist, oder nicht. Dies ermöglicht eine einfache visuelle Kontrolle des Zustands des Instruments. Weiterhin hat die helixartige Gestaltung der Führungsbahn den Vorteil, dass sich damit zum Betätigen der Unterdruckeinrichtung eine Schub-/Schraubbewegung ergibt. Sie ist ergonomisch günstiger als eine reine Schubbewegung, wie sie bei dem Instrument gemäß dem zuletzt genannten Stand der Technik erforderlich ist. Weiter kann durch die Wahl der Steigung der helixartigen Führungsbahn ein Übersetzungsverhältnis bestimmt werden. Mit einer steilen Steigung kann ein verhältnismäßig großer Unterdruck aufgebaut werden, wohingegen mit einer geringeren Steigung die zum Erreichen des Unterdrucks erforderlichen Betätigungskräfte reduziert werden können. In beiden Fällen bewirkt ein einfaches Fortsetzen der Bewegung, dass das Betätigungsorgan am Ende in die Arretierposition gelangt. Damit vereinfacht das erfindungsgemäße Instrument nicht nur die Handhabung, sondern erhöht auch die Betätigungs- und damit auch die Haltesicherheit. Ein weiterer Vorteil liegt darin, dass zum Abnehmen des Instruments kein gesondertes Belüftungsventil erforderlich ist. Es genügt vielmehr, das Betätigungsorgan über einen gewissen Widerstand hinweg aus seiner Arretierposition zu bewegen, und entlang der helixartig geformten Führungsbahn wieder nach vorne zu bringen. Dadurch wird der Unterdruck automatisch abgebaut, wodurch das Instrument leicht von der eingesetzten Gelenkpfanne abgenommen werden kann. Die Saugeinrichtung beruht damit nur noch ein bewegtes Teil aufzuweisen, nämlich den Kolben. Dank des Verzichts auf ein gesondertes Belüftungsventil ist damit die Herstellung einfacher, und die Betriebssicherheit erhöht. Undichtigkeiten am Belüftungsventil können Dank der Erfindung nicht mehr zu einem schleichenden Verlusts des Vakuums führen. Damit bietet das erfindungsgemäße Instrument nicht nur eine bessere Haltbarkeit, sondern auch eine größere Sicherheit bei geringerem Herstellungsaufwand.

Zweckmäßig ist es, wenn am hinteren Ende des Schafts ein auswechselbarer Führungskopf vorgesehen ist, an dem die Führungsbahn angeordnet ist. Dies ermöglicht es, den Führungskopf zu Reinigungszwecken abzunehmen oder gegen einen anderen auszutauschen. Bei abgenommen Führungskopf kann der Kolben der Saugeinrichtung leicht nach hinten aus dem Schaft entnommen werden. Das Instrument kann damit auf einfache Weise in seine Einzelteile zerlegt und sicher gereinigt werden. Durch Bereitstellen weiterer Führungsköpfe mit unterschiedlich geformten Führungsbahnen, insbesondere solcher mit größerer oder kleinerer Steigung, kann das Instrument an verschiedene Einsatzszenarien angepasst werden. So können beispielsweise zum Haltern von verhältnismäßig schweren Gelenkpfannen Führungsköpfe mit geringerer Steigung vorgesehenen sein, um bei gleicher Betätigungskraft ein größeres Vakuum aufzubauen. Umgekehrt kann bei kleineren leichten Gelenkpfannen eine steilere Führungsbahn vorgesehen sein, um eine schnelle Betätigung zu ermöglichen.

Zweckmäßigerweise ist an dem hinteren Ende des Schafts ein Schlagkopf ausgebildet. Dieser kann schaftfest oder an den auswechselbaren Führungskopf vorgesehen sein. Er wirkt als eine Art Amboss, um beim Einsetzen der Gelenkpfanne mit einem Schlagwerkzeug auf das Instrument einwirken zu können. Dank der erfindungsgemäßen Arretierung des Betätigungsorgans ist sichergestellt, dass sich unter der Schlageinwirkung das Betätigungsorgan nicht aus der Saugposition löst, wodurch ein unbeabsichtigter Verlust des Vakuums sicher verhindert wird.

Der Saugkopf weist eine umlaufende Anlagefläche auf, an welchem die von dem Instrument gehaltene Gelenkpfanne anliegt. Die Anlagefläche dient insbesondere auch zur Übertragung von Einsetzkräften, wie beim Einschlagen. Um auch unter Schlageinwirkungen eine ausreichende Abdichtung zu haben, damit ein unerwünschtes Entweichen des Vakuums zwischen Saugkopf und Gelenkpfanne zu verhindern, ist zweckmäßigerweise ein Dichtring vorgesehen. Dieser ist zwischen Anlagefläche und Mündung des Saugkanals angeordnet. Er kann als ein O-Ring ausgeführt und einfach oder auch doppelt bzw. mehrfach vorgesehen sein. Zweckmäßigerweise ist er auswechselbar, um im Fall von Verschleiß gegen einen anderen ausgetauscht werden.

Um eine Beschädigung der empfindlichen Gelenkpfanne bzw. des Keramik-Einsatzes zu vermeiden, ist vorzugsweise ein Schutzring für die Anlagefläche des Saugkopfs vorgesehen. Dieser kann integriert oder gesondert ausgeführt sein. Er ist zweckmäßiger Weise aus einem Kunststoffmaterial hergestellt. Er vermeidet einen direkten Kontakt zwischen der Anlagefläche des Saugkopfs und der Gegenfläche der Gelenkpfanne bzw. des Einsatzes. Der Gefahr von Beschädigungen, wie sie insbesondere bei einem aus Metall ausgeführten Saugkopf und einem aus Keramik bestehenden Einsatz auftreten würden, kann damit auf wirksame Weise begegnet werden. Der Schutzring ist aber nicht nur eine mechanisch schützende Funktion, sondern kann darüber hinaus auch noch zusätzlich als weitere Abdichtung wirken.

An dem Saugkopf kann weiter eine Führungsfläche vorgesehen sein. Sie liegt zwischen Anlagefläche und Mündung der Saugleitung und ist vorzugsweise kegelig oder sphärisch ausgestaltet. Sie vereinfacht das Einführen des Saugkopfes in die Öffnung der Gelenkpfanne bzw. des Einsatzes. Der Gefahr von Beschädigungen auf Grund von Ungenauigkeiten beim Ansetzen der Gelenkpfanne bzw. des Einsatzes an den Saugkopf wird damit entgegen gewirkt.

Vorzugsweise ist der Saugkopf auswechselbar ausgeführt. Mit Vorteil sind mehrere Saugköpfe für verschiedene Größen von Gelenkpfannen bzw. Einsätzen vorgesehen ist. Damit kann ein Instrumentarium geschaffen werden, das es ermöglicht, Gelenkpfannen bzw. Einsätze unterschiedlicher Größe und Form mit dem erfindungsgemäßen Instrument zu handhaben. Es genügt, wenn das erfindungsgemäße Instrument lediglich einmal vorhanden ist und der jeweils verwendete Saugkopf entsprechend dem einzusetzenden Einsatz bzw. der einzusetzenden Pfanne ausgewählt ist. Dabei können außer einem Satz von Saugköpfen für verschiedene Größen noch Zusatzsätze vorgesehen sein, welche Prothesen bzw. Einsätze anderen Typs abdecken.

Die Erfindung wird nachfolgend unter Bezugnahme auf die Zeichnung erläutert, welche ein vorteilhaftes Ausführungsbeispiel darstellt. Es zeigen:

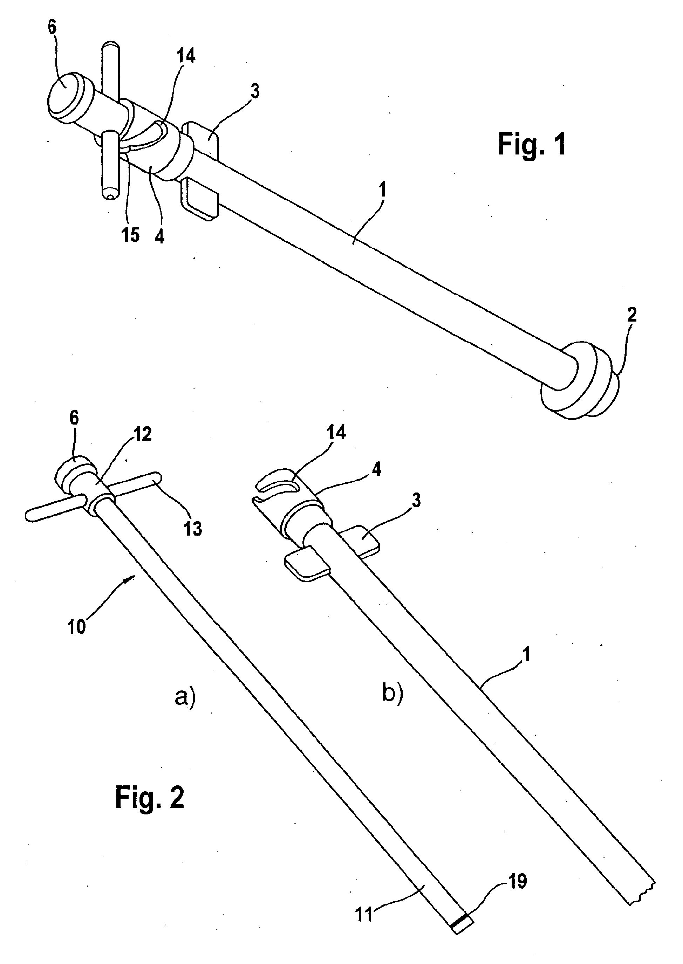

Fig. 1 eine perspektivische Ansicht eines Ausführungsbeispiels des erfindungsgemäßen Instruments;
Fig. 2a und b einen Schaft und einen Kolben der Saugeinrichtung mit Betätigungsorgan des in Fig. 1 abgebildeten Instruments;
Fig. 3a und b zwei verschiedene Saugköpfe zur Montage am Schaft des Instruments; und
Fig. 4a und b das Betätigungsorgan der Saugeinrichtung in einer Ruhe- und Saugposition.

Das erfindungsgemäße Instrument umfasst im Wesentlichen einen zylinderartig gestreckt ausgebildeten Schaft 1. An seinem vorderen Ende ist ein Saugkopf 2 auswechselbar mittels einer Gewindeverbindung aufgeschraubt. Am hinteren Ende des Schafts 1 befindet sich ein Flügelpaar 3, welches aus zwei diametral gegenüberliegend angeordneten, radial nach außen abstehenden Flügeln als Handgriff besteht. Weiter nach hinten schließt sich ein ebenfalls auf den Schaft aufschraubbarer Führungskopf 4 an.

Der Schaft 1 ist als Hohlkörper ausgefüllt und weist einen zylindrischen Hohlraum (nicht dargestellt) auf. Er ist Bestandteil einer Saugeinrichtung 10, die nachfolgend noch näher erläutert wird. Der Hohlraum ist über einen Saugkanal 17 in dem Saugkopf 2 verbunden mit der Spitze des Saugkopfes 2. Der Saugkanal 17 endet dort mit einer Mündung 18. Der Saugkopf 2 weist weiter als Hauptkomponente einen Anlagebund 21 und einen zylindrisch geformten Fortsatz 22 mit einer umlaufenden Rille 23 auf. Der Anlagepunkt dringt mit seiner rückseitigen, dem Schaft 1 zugewandten Fläche als Anschlag für eine Schraubverbindung 29, mit welcher der Saugkopf 2 an das vordere Ende des Schaftes 1 geschraubt ist. An der vorderen Stirnfläche des Anschlagbunds 21 ist eine Anlagefläche 26 ausgebildet. Sie dient dazu, mit einer Stirnfläche eines Keramikeinsatzes (nicht dargestellt) einer Hüftprothese zusammen zu wirken, um Schubkräfte zum Einsetzen des Keramikeinsatzes in eine Lagerpfanne (nicht dargestellt) auszuüben. Zur Schonung und zur besseren Abdichtung ist ein Schutzring 27 vorgesehen. Dieser weist etwa die Abmessungen der Anlagefläche 26 auf und liegt zum Schutz der Oberflächen zwischen der Anlagefläche 26 und der als Gegenfläche fungierenden Stirnfläche des Keramikeinsatzes (nicht dargestellt). Der Durchmesser des zylindrischen Fortsatzes 22 ist so auf die lichte Weite der Öffnung des Keramikeinsatzes abgestimmt, dass der Saugkopf 2 mit Spiel in den Keramikeinsatz zentrisch einführbar ist. Ist der Saugkopf 2 soweit eingebracht, dass die Stirnfläche des Keramikeinsatzes an der Anlagefläche 26 bzw. dem Schutzring 27 anliegt, so kommt ein in der Rille 23 eingesetzter Dichtring 28 in Kontakt mit der Innenseite des Keramikeinsatzes (nicht dargestellt). Dadurch ergibt sich eine Abdichtung, sodass der vor dem Dichtring 28 liegende Bereich des Saugkopfes 2 mit der Mündung 18 bei aufgesetztem Keramikeinsatz gegenüber der Umgebung abgedichtet ist. Dadurch kann der Keramikeinsatz an den Saugkopf 2 gehalten werden.

Aufbau und Funktionsweise zu der Saugvorrichtung 10 werden nachfolgend erläutert. Sie umfasst im Wesentlichen einen Kolben 11, der mit einer Dichtung 19 an seinem vorderen Ende dichtend in den zylindrischen Hohlraum des Schaftes 10 eingeführt ist. In dem hinteren Bereich des Kolbens 11 ist ein Betätigungsorgan 12 mit einem als Querstab ausgeführten Handgriff 13 angeordnet. Das hintere Ende des Kolbens 11 ist als Schlagkopf 5 mit einem ambossartigen Aufsatz ausgebildet. Der in den Schaft 1 eingeführte Kolben 11 findet sich in Ruhe in der vorderen Position. Zum Betätigen der Saugeinrichtung 10 wird der Kolben 11 von Hand über den Handgriff 13 zurückgezogen. Der sich zurückbewegende Kolben 11 erzeugt damit in dem Saugkanal 17 und damit in dem Bereich zwischen der Dichtung 28 und dem Keramikeinsatz einen Unterdruck. Dadurch ist der Keramikeinsatz fest an dem Saugkopf 2 gehaltert, solange der Unterdruck aufrecht gehalten bleibt. Soll das Instrument wieder abgenommen werden, so wird der Kolben 11 mittels des Betätigungsorgans 12 wieder nach vorne bewegt, wodurch der Unterdruck abgebaut wird, und der Saugkopf sich leicht von dem Keramikeinsatz abnehmen lässt.

Ein wesentliches Merkmal der Erfindung liegt in der Führung des Betätigungsorgans 12 an dem Schaft 10. Schaft 10 weist an seinem hinteren Ende den als eine Helixhülse ausgebildeten Führungskopf 4 auf. An ihm sind zwei sich schraubenförmig in Umfangsrichtung schräg nach vorne erstreckende Führungsbahnen 14 ausgebildet. Sie weisen in ihrem hinteren Bereich einen Absatz mit einer Vertiefung 15, und in ihrem vorderen Bereich einen Steigungsabschicht 16 auf. Der Steigungsabschnitt 16 ist so ausgebildet, dass seine Abwicklung eine Gerade mit einem bestimmten Steigungswinkel bildet. Die Weite der Führungsbahn 15 ist so bemessen, dass der Querstab des Handgriffs 13 darin nahezu spielfrei geführt ist. Das vordere Ende der Führungsbahn 14 definiert die vordere Anschlagposition des Handgriffs 13 und damit auch die des Kolbens 11. Dies ist die Ruheposition. Aus dieser kann der Kolben über eine Schub-/Schraubbewegung am Handgriff 13 nach hinten bewegt werden, bis schließlich der Handgriff 13 über einen Absatz seitlich in die Vertiefung 15 gleitet, und dort arretiert. Dies definiert die Saugposition. Um dem Operateur die Ausführung der Schub-/Schraubbewegung zu erleichtern, sind die Flügel 3 an dem Schaft 10 vorgesehen. Dadurch kann der Operateur das Instrument mit der einen Hand am Schaft 1 halten und über die Flügel 3 so abstützen, dass eine ungewollte Verdrehung bei dem Betätigen der Saugeinrichtung 10 am Handgriff 13 verhindert wird. Der Steigungswinkel des Steigungsabschnitts 16 bestimmt das Verhältnis von Schubzu Schraubbewegung. Soll das Betätigungsorgan 12 mit verhältnismäßig geringer Kraft betätigbar sein, so ist der Winkel zweckmäßigerweise klein zu wählen. Das heißt, der Steigungsabschnitt 16 verläuft mehr zur Seite hin als nach vorne. Sind hingegen keine großen Bestätigungskräfte zu erwarten, so kann es aus Gründen einer leichteren und schnelleren Betätigung zweckmäßig sein, den Steigungsabschnitt 16 steil auszuführen, also mit einem großen Steigungswinkel zu versehen. Damit ergibt sich dann bei geringer Schraubbewegung des Kolbens 11 ein verhältnismäßig großer Hub. Die Betätigungsgeschwindigkeit erhöht sich dadurch, allerdings sind dafür höhere Betätigungskräfte erforderlich.

Hat die Saugeinrichtung 10 die Saugposition erreicht, so ist der Handgriff 13 in der Vertiefung 15 der Führungsbahn 14 arretiert. Damit ist der Handgriff 13 gegenüber einem unbeabsichtigten Herausspringen aus der Arretierungsposition gehindert. Durch die Anlage des Handgriffs 13 in der Vertiefung 15 der Führungsbahn 14 ist weiter eine Kraftschlüssige Verbindung in Axialrichtung zwischen dem Kolben 11 mit dem Schlagkopf 6 an seinem hinteren Ende und dem Schaft 1 und seiner Anlagefläche 26 Ende geschaffen. Damit kann der Impuls von Schlageinwirkungen auf dem Schlagkopf 6 über den Haltegriff 13, die Vertiefung 15, den Schaft 10 auf die Anlagefläche 26 und damit schließlich auf den einzusetzenden Keramikeinsatz übertragen werden. Dank der sicheren Arretierung des Handgriffs 13 in der Vertiefung 15 ist ein Herausspringen der Betätigungseinrichtung 12 aus der Saügposition nicht zu befürchten. Nach dem Einschlagen des Keramikeinsatzes kann durch einfaches Verdrehen des Handgriffs 13 der Kolben 11 wieder in die Ruheposition zurückgebracht werden, wodurch der Unterdruck entweicht und der Saugkopf 2 leicht von dem Keramikeinsatz abgenommen werden kann.

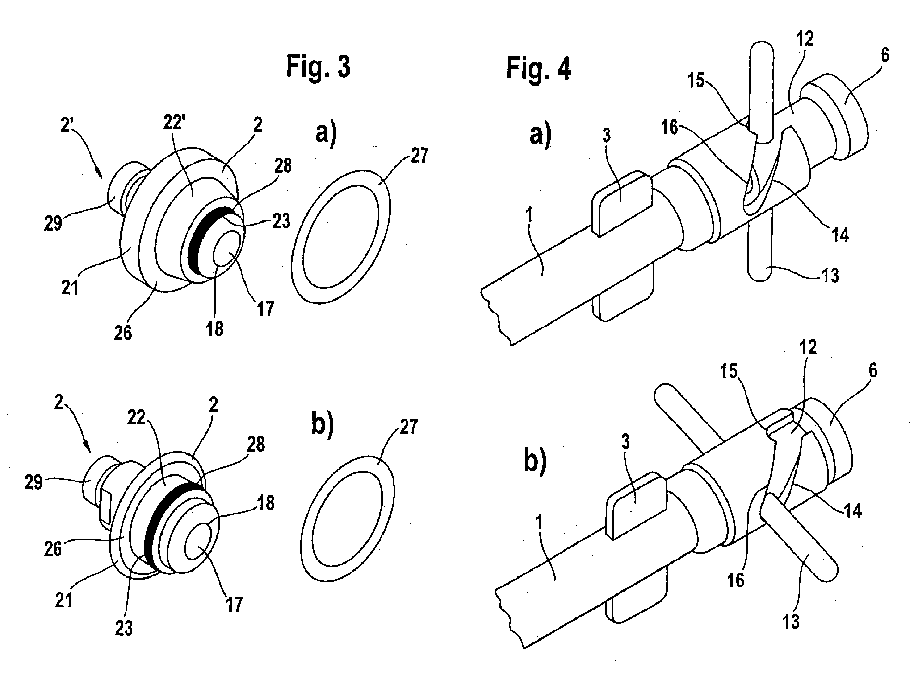

Der Saugkopf 2 ist, wie bereits eingangs erläutert, schraubbar an dem Schaft 10 befestigt. Mit Vorteil ist ein Satz von Saugköpfen vorgesehen, der neben dem Saugkopf 2 auch weitere Saugköpfe 2' in anderer Größe oder mit anderer Formgebung umfasst. In Figur 3a ist ein Saugkopf 2' dargestellt, der für einen größeren Keramikeinsatz mit anderer Gestaltung der Pfannenform ausgebildet ist. Er unterscheidet sich von dem in Figur 3b dargestellten im Wesentlichen dadurch, dass die Rille zur Aufnahme des Dichtrings in einem sphärisch statt zylindrisch geformten Fortsatz 22' angeordnet ist. Es versteht sich, dass weitere alternative Saugköpfe in anderen Formen und/oder Größen vorgesehen sein können.

## Patentansprüche

1. Einsetzinstrument für Gelenkpfannen von Prothesen mit einem lang gestrecktem Schaft (1), an dessen vorderem Ende sich ein Saugkopf (2, 2') zur Verbindung mit der Gelenkpfanne befindet, mit einer Saugeinrichtung (10) mit einem im Schaft (1) geführten Kolben (11), mit einer am Saugkopf (2, 2') mündenden Saugleitung (17) und mit einem Betätigungsorgan (12), das entlang einer Führungsbahn (14) an dem Schaft (1) geführt ist, wobei die Führungbahn eine Arretierungseinrichtung für das Betätigungorgan in einer Saugposition aufweist,
**dadurch gekennzeichnet dass**,
die Führungsbahn (14) helixartig geformt ist.

2. Einsetzinstrument nach Anspruch 1,
**dadurch gekennzeichnet dass**,
die Arretierungseinrichtung als eine Vertiefung (15) ausgeführt.

3. Einsetzinstrument nach Anspruch 1 oder 2,
**dadurch gekennzeichnet dass**,
am hinteren Ende des Schafts (10) ein auswechselbarer Führungskopf (4) vorgesehen ist, an dem die Führungsbahn (14) angeordnet ist.

4. Einsetzinstrument nach Anspruch 2 und 3,
**dadurch gekennzeichnet dass**,
ein Schlagkopf (6) an dem Betätigungsorgan (12) ausgebildet ist.

5. Einsetzinstrument nach einem der vorangehenden Ansprüche,
**dadurch gekennzeichnet dass**,
an dem Saugkopf (2, 2') eine zwischen Anlagefläche (26) und Mündung (18) angeordnete Dichtung (28) vorgesehen ist.

6. Einsetzinstrument nach Anspruch 5,
**dadurch gekennzeichnet dass**,
die Dichtung (28) auswechselbar ist.

7. Einsetzinstrument nach einem der vorangehenden Ansprüche
**dadurch gekennzeichnet dass**,
ein Schutzring (27) vorgesehen ist, der die Anlagefläche (26) bedeckt.

8. Einsatzinstrument nach einem der vorangehenden Ansprüche,
**dadurch gekennzeichnet, dass**
der Saugkopf (2, 2,') zusätzlich eine Führungsfläche aufweist.

9. Einsetzinstrument nach Anspruch 8,
**dadurch gekennzeichnet dass**,
die Führungsfläche (22') sphärisch ausgebildet ist.

10. Einsetzinstrument nach Anspruch 8,
**dadurch gekennzeichnet dass**,
die Führungsfläche zylindrisch (22) ausgebildet ist.

11. Einsetzinstrument nach einem der vorhergehenden Ansprüche,
**dadurch gekennzeichnet, dass**
mehrere Einsätze von Saugköpfen (2, 2') in verschiedenen Größen vorgesehen sind.

12. Einsetzinstrument nach einem der vorhergehenden Ansprüche,
**dadurch gekennzeichnet dass**,
ein Zusatz Satz Saugköpfe (2') für einen anderen Typ von Gelenkpfannen vorgesehen ist.

## Claims

1. Insertion instrument for joint sockets of prostheses, with an elongate shaft (1), a suction head (2, 2') being located at the front end thereof for connection to the joint socket, with a suction device (10) with a piston (11) guided in the shaft (1), with a suction line (17) opening out at the suction head (2, 2'), and with an actuating element (12) which is guided along a guide track (14) on the shaft (1), wherein the guide track has a locking arrangement for the actuating element in a suction position, **characterized in that** the guide track (14) is helically shaped.

2. Insertion instrument according to Claim 1, **characterized in that** the locking arrangement is designed as a recess (15).

3. Insertion instrument according to Claim 1 or 2, **characterized in that** the rear end of the shaft (1) is provided with an exchangeable guide head (4) on which the guide track (14) is arranged.

4. Insertion instrument according to Claims 2 and 3, **characterized in that** a strike head (6) is formed on the actuating element (12).

5. Insertion instrument according to one of the preceding claims, **characterized in that** a seal (28) arranged between bearing surface (26) and mouth (18) is provided on the suction head (2, 2').

6. Insertion instrument according to Claim 5, **characterized in that** the seal (28) is exchangeable.

7. Insertion instrument according to one of the preceding claims, **characterized in that** a protective ring (27) is provided which covers the bearing surface (26).

8. Insertion instrument according to one of the preceding claims, **characterized in that** the suction head (2, 2') additionally has a guide surface.

9. Insertion instrument according to Claim 8, **characterized in that** the guide surface (22') is spherically shaped.

10. Insertion instrument according to Claim 8, **characterized in that** the guide surface is cylindrically shaped (22) .

11. Insertion instrument according to one of the preceding claims, **characterized in that** several sets of suction heads (2, 2') in different sizes are provided.

12. Insertion instrument according to one of the preceding claims, **characterized in that** an additional set of suction heads (2') is provided for another type of joint socket.

## Revendications

1. Instrument de pose pour les cavités glénoïdes de prothèses, comportant une tige (1) allongée, à l'extrémité avant de laquelle se situe une tête aspirante (2, 2') destinée à être reliée à la cavité glénoïde, et comportant un dispositif d'aspiration (10) avec un piston (11) logé dans la tige (1), comportant une conduite d'aspiration (17) débouchant au niveau de la tête aspirante (2, 2'), et comportant un organe d'actionnement (12) qui est guidé le long d'une voie de guidage (14) sur la tige (1), ladite voie de guidage comportant un dispositif d'immobilisation pour l'organe d'actionnement dans une position d'aspiration, **caractérisé en ce que** la voie de guidage (14) a une forme hélicoïdale.

2. Instrument de pose selon la revendication 1, **caractérisé en ce que** le dispositif d'immobilisation est réalisé sous la forme d'un creux (15).

3. Instrument de pose selon la revendication 1 ou 2, **caractérisé en ce qu'**au niveau de l'extrémité arrière de la tige (1) est prévue une tête de guidage (4) remplaçable, sur laquelle est disposée la voie de guidage (14).

4. Instrument de pose selon les revendications 2 et 3, **caractérisé en ce qu'**une tête de percussion (6) est réalisée sur l'organe d'actionnement (12).

5. Instrument de pose selon l'une quelconque des revendications précédentes, **caractérisé en ce qu'**un joint d'étanchéité (28), disposé entre la surface d'appui (26) et la bouche (18), est prévu sur la tête aspirante (2, 2').

6. Instrument de pose selon la revendication 5, **caractérisé en ce que** le joint d'étanchéité (28) est remplaçable.

7. Instrument de pose selon l'une quelconque des revendications précédentes, **caractérisé en ce qu'**il est prévu une bague de protection (27) qui recouvre la surface d'appui (26).

8. Instrument de pose selon l'une quelconque des revendications précédentes, **caractérisé en ce que** la tête aspirante (2, 2') comporte, en plus, une surface de guidage.

9. Instrument de pose selon la revendication 8, **caractérisé en ce que** la surface de guidage (22') est sphérique.

10. Instrument de pose selon la revendication 8, **caractérisé en ce que** la surface de guidage (22) est cylindrique.

11. Instrument de pose selon l'une quelconque des revendications précédentes, **caractérisé en ce qu'**il est prévu plusieurs jeux de têtes aspirantes (2, 2') de tailles différentes.

12. Instrument de pose selon l'une quelconque des revendications précédentes, **caractérisé en ce qu'**il est prévu un jeu supplémentaire de têtes aspirantes (2') pour un autre type de cavités glénoïdes.
